**Europäisches Patentamt**

**European Patent Office**

**Office européen des brevets**

(19)

(11) Numéro de publication: **0 170 602 B1**

## FASCICULE DE BREVET EUROPÉEN

(12)

(45) Date de publication du fascicule du brevet:
02.08.89

(51) Int. Cl.⁴: **A 61 L 2/20,** G 02 C 13/00

(21) Numéro de dépôt: **85440037.1**

(22) Date de dépôt: **30.05.85**

(54) **Procédé de décontamination et de nettoyage de prothèses oculaires, en particulier de lentilles de contact, et dispositif pour la mise en oeuvre de ce procédé.**

(30) Priorité: **04.07.84 FR 8410728**

(43) Date de publication de la demande:
**05.02.86 Bulletin 86/6**

(45) Mention de la délivrance du brevet:
**02.08.89 Bulletin 89/31**

(84) Etats contractants désignés:
**AT BE CH DE GB IT LI LU NL SE**

(56) Documents cité:
**EP-A-0 079 827**
**WO-A-83/00447**
**FR-A-2 492 665**
**US-A-3 473 886**
**US-A-3 852 032**

(73) Titulaire: **Nold, Yves, 9, rue du Parc, F-57100 Thionville (FR)**

(72) Inventeur: **Arnold, Noel, 105, rue de la Semm, F-68000 Colmar (FR)**

(74) Mandataire: **Nuss, Pierre, 10, rue Jacques Kablé, F-67000 Strasbourg (FR)**

EP 0 170 602 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

**Description**

La présente invention concerne le domaine des dispositifs de correction de vue, en particulier des lentilles de contact, et a pour objet un procédé de décontamination et de nettoyage de prothèses oculaires, en particulier de lentilles de contact.

L'invention a également pour objet un dispositif pour la mise en oeuvre de ce procédé.

L'oeil humain équipé de lentilles de contact, doit obligatoirement être protégé de l'infection microbienne. Un des problèmes associé à ces lentilles, réside dans leur produit d'entretien. Celui-ci requiert, outre une simplicité d'utilisation, des qualités d'efficacité microbiologique et des propriétés nettoyantes.

A cet effet, il existe plusieurs procédés qui sont regroupés selon leur mode d'action en deux catégories, à savoir chimique et physique.

Ces procédés présentent cependant certains inconvénients.

Les procédés chimiques mettent en oeuvre des solutions isotoniques et stérilisantes contenant des conservateurs tels le gluconate de chlorhexidine, des dérivés mercuriels ou des ammoniums quaternaires. L'emploi de ces solutions exige de nombreuses opérations contraignantes pour l'utilisateur. En outre, les réactions de toxicité et d'irritation oculaires ne sont pas rares.

Un deuxième procédé consiste à traiter les lentilles avec une solution à 3 % d'eau oxygénée. Ce procédé est efficace du point de vue de la destruction des germes, mais la solution utilisée doit être neutralisée par un agent réducteur sous peine de provoque de vives brûlures à l'oeil de l'utilisateur.

Dans la catégorie des procédés physiques, on peut citer l'ébullition, qui est un procédé confirmé pour la stérilisation de l'eau, mais qui présente l'inconvénient, dans le cas des lentillès de contact, de provoquer une lente opacification due à la dénaturation du matériel muco-protéique.

On connaît également, par US-A-3 852 032, un procédé de stérilisation de lentilles de contact faisant intervenir le rayonnement ultra-violet.

Cependant, ce procédé connu présente un risque de dégradation des lentilles par ledit rayonnement ultra-violet nécessitant la disposition des lentilles dans un gel hydrophile, qui a pour conséquence de réduire de manière significative l'action germicide dudit rayonnement.

Il a également été proposé, par US-A-3 473 886 un appareil médical de stérilisation de lentilles de contact, dans lequel un gaz stérilisant est injecté sous pression dans un récipient de réception des lentilles à traiter. Dans un tel appareil, il n'est prévu aucun moyen de protection des lentilles contre des effets secondaires nocifs du gaz de traitement.

Il n'existe donc, à l'heure actuelle, aucun procédé réunissant simplicité d'utilisation et absence d'effets secondaires indésirables.

La présente invention a pour but de pallier ces inconvénients.

Elle a, en effet, pour objet un procédé de décontamination et de nettoyage de prothèses oculaires, en particulier de lentilles de contact, caractérisé en ce qu'il consiste essentiellement à produire de l'ozone par ionisation dans un tube ionisant, à faire migrer cet ozone à travers une membrane filtrante et ensuite à faire diffuser ledit ozone dans une solution isotonique de trempage des lentilles pendant une durée prédéterminée, de préférence de l'ordre de deux heures.

L'invention a également pour objet un dispositif pour la mise en oeuvre de ce procédé, qui est caractérisé en ce qu'il est essentiellement constitué par un boîtier comportant un tableau de commande muni d'un interrupteur de mise en marche et d'arrêt, par un voyant lumineux, par un récipient amovible de réception des prothèses oculaires et renfermant une solution isotonique dans laquelle diffuse de l'ozone pénétrant à travers une membrane filtrante et issu d'un tube ionisant monté dans un corps cylindrique fixé de manière amovible au tableau de commande du boîtier, ledit tube ionisant étant commandé par un circuit électrique de commande.

L'invention sera mieux comprise grâce à la description ci-après, qui se rapporte à un mode de réalisation préféré, donné à titre d'exemple non limitatif, et expliqué avec référence aux dessins schématiques annexés, dans lesquels:

la figure 1 est une vue en perspective d'un dispositif conforme à l'invention;

la figure 2 est une vue en coupe, et à plus grande échelle, du dispositif suivant la figure 1;

les figures 3 et 4 sont des vues en coupe de deux modes de réalisation possibles du dispositif de filtration, et

la figure 5 est un schéma du circuit électrique de commande.

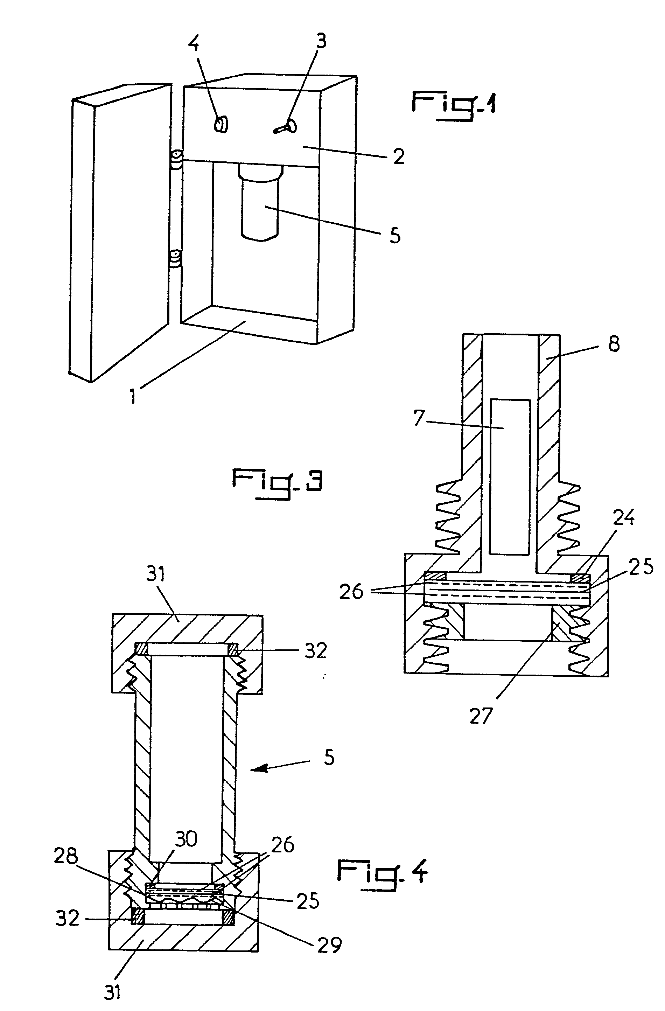

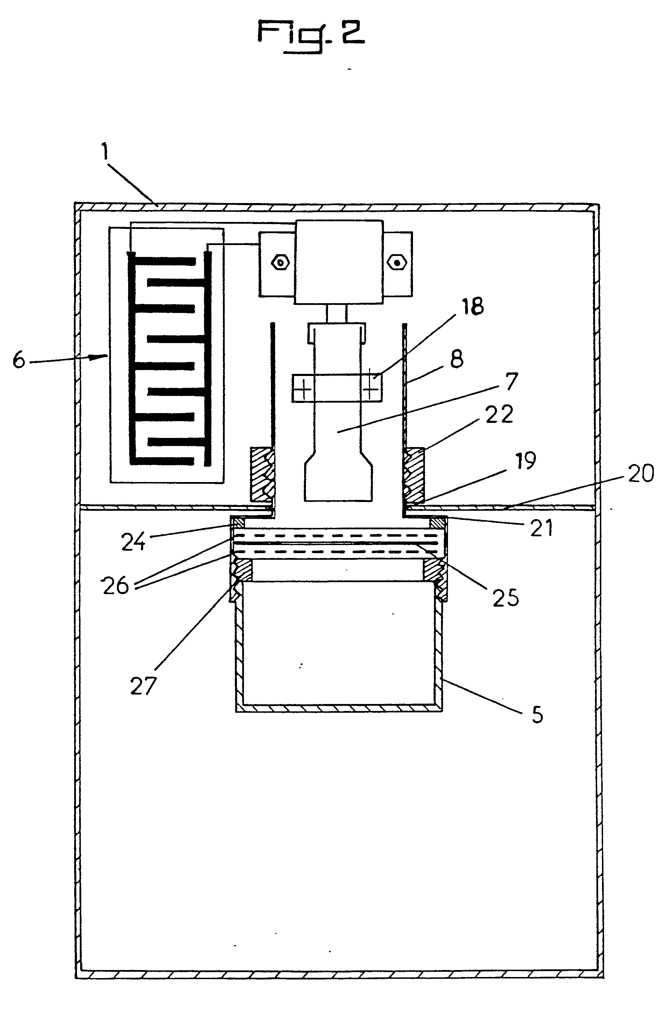

Conformément à l'invention, et comme le montrent plus particulièrement, à titre d'exemple, les figures 1 et 2 des dessins annexés, le dispositif pour la mise en oeuvre du procédé de décontamination et de nettoyage de prothèses oculaires, en particulier de lentilles de contact, est essentiellement constitué par un boîtier 1 qui comporte un tableau de commande 2 muni d'un interrupteur 3 de mise en marche et d'arrêt, par un voyant 4 formant témoin de fonctionnement du dispositif, par un récipient amovible 5 de réception des prothèses oculaires fixé sous le tableau de commande 2, et par un circuit électrique 6 de commande pour un tube ionisant 7, qui est monté dans un corps cylindrique 8, qui est fixé de manière amovible au tableau de commande 2 du boîtier 1.

Sur sa face arrière, il est pourvu de bornes de raccordement du circuit électrique 6 à une source de courant électrique, le circuit électrique 6 comprenant un transistor 9, deux condensateurs 10 et 11, une diode 12, une diode Zener 13, et trois résistances 14 à 16, et alimentent le tube

ionisant 7 par l'intermédiaire d'un transformateur 17.

L'alimentation électrique peut être à courant continu ou à courant alternatif.

Le corps cylindrique 8 de logement du tube ionisant 7, qui y est fixé au moyen de pattes de fixation 18, est réalisé, de préférence, en matière synthétique et ledit corps cylindrique 8 est engagé dans un orifice 19 de la face 20 correspondante du tableau de commande 2 jusqu'à une butée d'une partie élargie 21 dudit corps 8, un anneau de serrage 22 assurant le maintien du corps 8 dans l'orifice 19.

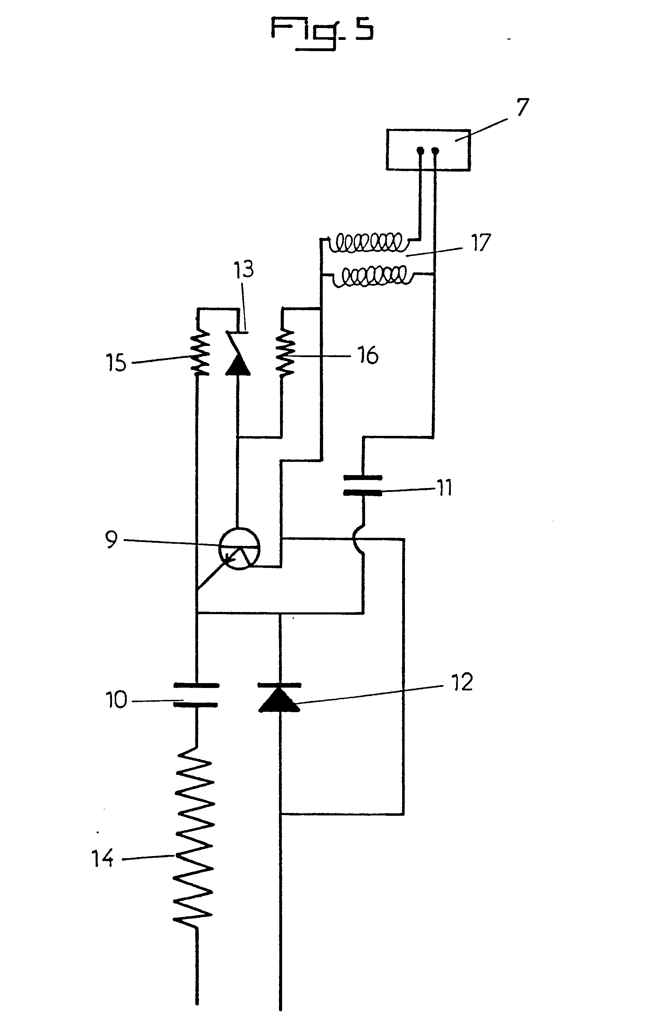

Le corps cylindrique 8 présente dans sa partie élargie 21 un filetage interne 23 permettant le serrage, au moyen du récipient 5, d'un dispositif de filtration constitué par un joint d'étanchéité 24 en matière chimiquement inerte, par une membrane filtrante 25 sélective aux gaz, et par des grilles de protection 26 disposées de part et d'autre de la membrane 25, un anneau de serrage 27 coopérant avec le filetage interne 23 assurant le serrage du dispositif (figure 3).

Le filetage 23 permet une fixation rapide du récipient 5 de réception des prothèses oculaires.

La membrane 25 est avantageusement une membrane ultra-mince, du type utilisé en microdialyse, présente des propriétés hydrophobes, et est fabriquée, de préférence, en silicone ou en PTFE (Polytétraflouréthylène).

Conformément à une variante de réalisation de l'invention, et comme le montre la figure 4, le dispositif de filtration est monté directement dans le récipient 5 de réception des prothèses oculaires, la membrane filtrante 25 et ses grilles de protection 26 étant maintenues dans leur logement 28 au moyen d'un anneau élastique 29, qui les plaque contre des lèvres de débordement 30.

Le récipient 5 est avantageusement muni à ses deux extrémités de bouchons à visser 31 dont l'étanchéité est assurée au moyen de joints toriques 32.

Pour le traitement des prothèses oculaires, l'un des bouchons 31 est retiré, et l'extrémité ainsi dégagée est fixée sur le filetage 23 du corps cylindrique 8 du dispositif par simple vissage.

Selon une autre caractéristique de l'invention, l'interrupteur 3 actionne une temporisation coupant l'alimentation électrique après une durée prédéterminée.

L'ozone utilisée dans le procédé conforme à l'invention est un agent chimique à l'état gazeux, qui, dans des conditions normales de pression et de température, est constitué de trois atomes d'oxygène, et qui s'auto-détruit en tendant à se dissocier en permanence d'après la transformation: $O_3 \rightarrow O_2 + O$.

Les atomes O s'unissent deux à deux pour former de l'oxygène $O_2$. De ce fait, l'ozone disparaît très rapidement lorsque s'arrête la source qui le génère, et il ne peut se produire d'effet secondaire lié à son absorption et à sa décharge ultérieure dans l'oeil de l'utilisateur.

Le dispositif conforme à l'invention a pour but de dissoudre l'ozone dans une solution isotonique de conservation des lentilles de contact, tout en isolant mécaniquement le milieu liquide contenant lesdites lentilles du générateur d'ozone, qui travaille en atmosphère sèche.

La concentration d'ozone dissous dans la solution isotonique est comprise entre 0,1 mg/l. et 10 mg/l. De préférence, la concentration d'ozone dissous dans la solution isotonique est de 1 mg/l.

En outre, le dispositif selon l'invention assure une préservation de toute contamination microbiologique après le traitement.

Le dispositif conforme à l'invention fonctionne de la manière suivante:

A titre d'exemple, il est fait référence à une opération de nettoyage et de décontamination de lentilles.

Dans un premier temps, un récipient 5 de réception de lentilles est fixé dans l'orifice de raccordement 23 du corps cylindrique 8 par vissage jusqu à obtention d'un verrouillage. Les bornes du dispositif sont alors raccordées au secteur, et ledit dispositif est mis en marche par actionnement de l'interrupteur 3, et le voyant 4 s'allume.

Le passage du courant dans le circuit électrique 6 alimente le tube ionisant 7, produisant ainsi de l'ozone, qui présente une densité supérieure à l'air ambiant, et qui descend dans le corps cylindrique 8 jusqu'au dispositif de filtration.

La différence de pression partielle entre les parois supérieure et inférieure de la membrane 25, provoque une migration d'ozone au travers de la membrane 25 et une diffusion dans le liquide de trempage des lentilles.

Après deux heures de fonctionnement, l'opération de nettoyage et de décontamination est terminée, et l'arrêt est effectué par actionnement inverse de l'interrupteur 3.

L'expérimentation a montré qu'une durée de traitement de deux heures était suffisante pour un nettoyage et une décontamination efficaces.

Les essais ont porté sur les germes suivants:

- <u>Escherichia coli</u>
- <u>Pseudomonas aeruginosa</u>
- <u>Staphylococcus aureus</u>
- <u>Candida albicans</u>

La densité de l'ensemencement dans la solution physiologique contenant les lentilles de contact, était de $10^6$ germes par ml.

Les résultats obtenus peuvent être résumés de la façon suivante:

Les microrganismes sont détruits dans des proportions qui diffèrent selon les espèces microbiennes.

Tous les germes sont tués dans le cas d'<u>Escherichia coli</u>, <u>Pseudomonas aeruginosa</u> et <u>Candida albicans</u>.

En ce qui concerne le <u>Staphylococcus aureus</u>, les bactéries sont détruites dans une proportion de 99, 99 %.

Il est à noter que la concentration de microorganismes choisie est nettement supérieure à celle rencontree avec des lentilles normalement contaminées.

Par ailleurs, l'efficacité du nettoyage sur une série de 20 lentilles usagées et présentant des dépôts colorés, a également été testée. Ce test a donné les résultats suivants:

- 10 lentilles sont réapparues claires et d'une parfaite transparence,
- 8 lentilles ont présenté une intensité de la coloration moindre, et
- 2 lentilles se sont avérées résistantes au traitement.

Enfin, des essais complémentaires ont démontré que 100 heures de traitement en continu sur différents types de lentilles, n'amènent aucune modification du spectre infrarouge due à une altération du matériau.

Le procédé et le dispositif conformes à l'invention permettent de réaliser le nettoyage et la décontamination de lentilles de contact de manière efficace et sans effet secondaire indésirable.

Le dispositif selon l'invention présente, en outre, l'avantage d'un faible encombrement et d'un faible prix de revient.

Bien entendu, l'invention n'est pas limitée au mode de réalisation décrit et représenté aux dessins annexés. Des modifications restent possibles, notamment du point de vue de la constitution des divers éléments, ou par substitution d'équivalents techniques, sans sortir pour autant du domaine de protection de l'invention.

**Revendications**

1. Procédé de décontamination et de nettoyage de prothèses oculaires, en particulier de lentilles de contact, caractérisé en ce qu'il consiste essentiellement à produire de l'ozone par ionisation dans un tube ionisant (7), a faire migrer cet ozone à travers une membrane filtrante (25), et ensuite à faire diffuser ledit ozone dans une solution isotonique de trempage des lentilles pendant une durée prédéterminée, de préférence de l'ordre de deux heures.

2. Procédé, suivant la revendication 1, caractérisé en ce que la concentration d'ozone dissous dans la solution isotonique est comprise entre 0,1 mg/l et 10 mg/l.

3. Procédé, suivant l'une quelconque des revendications 1 et 2, caractérisé en ce que la concentration d'ozone dissous dans la solution isotonique est, de préférence, de 1 mg/l.

4. Dispositif pour la mise en oeuvre du procédé suivant l'une quelconque des revendications 1 à 3, caractérisé en ce qu'il est essentiellement constitué par un boîtier (1) comportant un tableau de commande (2) muni d'un interrupteur (3) de mise en marche et d'arrêt, par un voyant lumineux (4), par un récipient amovible (5) de réception des prothèses oculaires et renfermant une solution isotonique dans laquelle diffuse de l'ozone pénétrant à travers une membrane filtrante (25) et issu d'un tube ionisant (7) monté dans un corps cylindrique (8) fixé de manière amovible au tableau de commande (2) du boîtier (1), ledit tube ionisant (7) étant commandé par un circuit électrique de commande (6).

5. Dispositif, suivant la revendication 4, caractérisé en ce que, sur sa face arrière il est pourvu de bornes de raccordement du circuit électrique (6) à une source de courant électrique, le circuit électrique comprenant un transistor (9), deux condensateurs (10 et 11), une diode (12), une diode Zener (13), et trois résistances (14 à 16), et alimentant le tube ionisant (7) par l'intermédiaire d'un transformateur (17).

6. Dispositif, suivant la revendication 4, caractérisé en ce que le corps cylindrique (8) de logement du tube ionisant (7), qui y est fixé au moyen de pattes de fixation (18), est réalisé, de préférence, en matière synthétique et ledit corps cylindrique (8) est engagé dans un orifice (19) de la face (20) correspondante du tableau de commande (2) jusqu'à une butée d'une partie élargie (21) dudit corps (8), un anneau de serrage (22) assurant le maintien du corps (8) dans l'orifice (19).

7. Dispositif, suivant l'une quelconque des revendications 4 et 6, caractérisé en ce que le corps cylindrique (8) présente dans sa partie élargie (21) un filetage interne (23) permettant le serrage, au moyen du récipient (5), d'un dispositif de filtration constitué par un joint d'étanchéité (24) en matière chimiquement inerte, par une membrane filtrante (25) sélective aux gaz, et par des grilles de protection (26) disposées de part et d'autre de la membrane (25), un anneau de serrage (27) coopérant avec le filetage interne (23) assurant le serrage du dispositif.

8. Dispositif, suivant la revendication 7, caractérisé en ce que la membrane (25) est avantageusement une membrane ultra-mince, du type utilisé en microdialyse, présente des propriétés hydrophobes, et est fabriquée, de préférence, en silicone ou en PTFE.

9. Dispositif, suivant l'une quelconque des revendications 7 et 8, caractérisé en ce que le dispositif de filtration est monté directement dans le récipient (5) de réception des prothèses oculaires, la membrane filtrante (25) et ses grilles de protection (26) étant maintenues dans leur logement (28) au moyen d'un anneau élastique (29), qui les plaque contre des lèvres de débordement (30).

10. Dispositif, suivant la revendication 4, caractérisé en ce que l'interrupteur (3) actionne une temporisation coupant l'alimentation électrique après une durée prédéterminée.

## Patentansprüche

1. Verfahren zur Entkeinung und Reinigung von Augenprothesen, insbesondere von Kontaktlinsen, dadurch gekennzeichnet, daß es im wesentlichen darin besteht, Ozon durch Ionisation in einer Ionenröhre (7) herzustellen, diesen Ozon durch eine Filtriermembran (25) migrieren zu lassen, und dann den Ozon in einer isotonischen Lösung zum Eintauchen der Linsen während einer bestimmten Dauer, vorzugsweise zwei Stunden lang diffundieren zu lassen.

2. Verfahren nach Anspruch 1, dadurch gekennzeichnet, daß die in der isotonischen Lösung diffundierte Ozonkonzentration zwischen 0,1 mg/l und 10 mg/l liegt.

3. Verfahren nach einem der Ansprüche 1 und 2, dadurch gekennzeichnet, daß die in der isotonischen Lösung diffundierte Ozonkonzentration vorzugsweise 1 mg/l beträgt.

4. Vorrichtung zur Durchführung des Verfahrens nach einem der Ansprüche 1 bis 3, dadurch gekennzeichnet, daß sie im wesentlichen aus einen Gehäuse (1), das ein mit einem Schalter (3) zum Ein- und Ausschalten versehenes Bedienungsfeld (2) aufweist, aus einer Leuchtanzeige (4) und aus einem abnehmbaren Behälter (5) zur Aufnahme der Augenprothesen besteht und eine isotonische Lösung enthält, in der Ozon diffundiert, der eine Filtriermembran (25) durchdringt und aus einer Ionisationsröhre (7) kommt, die in einem an dem Bedienungsfeld (2) des Gehäuses (1) abnehmbar befestigten, zylindrischen Körper (8) montiert ist, wobei die Ionisationsröhre (7) durch einen Stromkreis (6) betrieben wird.

5. Vorrichtung nach Anspruch 4, dadurch gekennzeichnet, daß sie an ihrer Rückseite mit Klemmen zum Anschluß des Stromkreises (6) an eine Stromquelle versehen ist, wobei der Stromkreis einen Transistor (9), zwei Kondensatoren (10 und 11), eine Diode (12), eine Zener-Diode (13) und drei Widerstände (14 bis 16) aufweist, und die Ionisationsröhre (7) mit Hilfe eines Transformators (17) speist.

6. Vorrichtung nach Anspruch 4, dadurch gekennzeichnet, daß der zylindrische Körper (8) zur Aufnahme der Ionisationsröhre (7), die daran mit Klammern (18) befestigt ist, vorzugsweise aus synthetischem Material hergestellt ist, und der zylindrische Körper (8) in einer Öffnung (19) der entsprechenden Seite (20) des Bedienungsfeldes (2) bis zu einem Anschlag einer Verbreiterung (21) des Körpers (8) eingesetzt ist, wobei ein Klemmring (22) die Halterung des Körpers (8) in der Öffnung (19) sichert.

7. Vorrichtung nach einem der Ansprüche 4 und 6, dadurch gekennzeichnet, daß der zylindrische Körper (8) an seiner Verbreiterung (21) ein Innengewinde (23) aufweist, das die Verklemmung, mit Hilfe des Behälters (5), einer Filtriereinrichtung ermöglicht, die aus einer Dichtung (24) aus chemisch neutralem Material, aus einer gegenüber Gas selektiven Filtriermembran (25), und aus zu beiden Seiten der Membran (25) angeordneten Schutzgittern (26) besteht, wobei ein mit dem Innengewinde (23) zusammenwirkender Klemmring (27) die Klemmung der Einrichtung sichert.

8. Vorrichtung nach Anspruch 7, dadurch gekennzeichnet, daß die Membran (25) vorteilhaft eine ultrafeine Membran ist, von dem Typ, wie sie bei der Microdialyse verwendet werden, daß sie hydrophobe Eigenschaften besitzt und vorzugsweise aus Silicon oder PTFE hergestellt ist.

9. Vorrichtung nach einem der Ansprüche 7 und 8, dadurch geennzeichnet, daß die Filtriereinrichtung direkt in den Behälter (5) zur Aufnahme der Augenprothesen montiert ist, wobei die Filtriermembran (25) und ihre Schutzgitter (26) in ihrem Sitz (28) durch einen elastischen Ring (29) gehalten werden, der sie gegen die Überlappungen (30) drückt.

10. Vorrichtung nach Anspruch 4, dadurch gekennzeichnet, daß der Schalter (3) ein Zeitrelais in Gang setzt, das die Stromzufuhr nach einer bestimmten Zeit abschaltet.

## Claims

1. Method of decontaminating and cleaning eye prostheses, in particular contact lenses, characterised in that it essentially involves producing ozone by ionisation in an ionising tube (7), causing this ozone to migrate through a membrane filter (25) and then causing said ozone to diffuse in an isotonic lens soaking solution for a predetermined period, preferably of about two hours.

2. Method according to claim 1, characterised in that the concentration of ozone dissolved in the isotonic solution is between 0.1 mg/l and 10 mg/l.

3. Method according to any one of claims 1 and 2, characterised in that the concentration of ozone dissolved in the Isotonic solution is preferably 1 mg/l.

4. Device for carrying out the method according to any one of claims 1 to 3, characterised in that it is essentially made up of a casing (1) comprising a control panel (2) equipped with a starting and stopping switch (3), an indicator light (4), a removable container (5) for receiving the eye prostheses and containing an isotonic solution in which there diffuses the ozone penetrating through a membrane filter (25) and issuing from an ionising tube (7) mounted in a cylindrical member (8) fixed removably on the control panel (2) of the casing (1), said ionising tube (7) being controlled by an electric control circuit (6).

5. Device according to claim 4, characterised in that, on its rear face, it is provided with terminals for connection of the electric circuit (6) to a source of electric current, the electric circuit comprising a transistor (9), two capacitors (10 and 11), a diode (12), a Zener diode (13), and three resistors (14 to 16), and supplying the ionising tube (7) via a transformer (17).

6. Device according to claim 4, characterised in that the cylindrical member (8) accommodating the ionising tube (7) which is fixed therein by means of fixing tabs (18) is preferably produced from synthetic material and said cylindrical member (8) is engaged in an orifice (19) in the corresponding face (20) of the control panel (2) up to an abutment of a widened portion (21) of said member (8), a gripping ring (22) ensuring that the member (8) is held in the orifice (19).

7. Device according to any one of claims 4 and 6, characterised in that the cylindrical member (8) has, in its widened portion (21), an internal thread (23) allowing the gripping, by means of the container (5), of a filtering device formed by a seal (24) of chemically inert material, by a membrane filter (25) which is selective to gases, and by protective grids (26) arranged on either side of the membrane (25), a gripping ring (27) which cooperates with the internal thread (23) allowing the device to be gripped.

8. Device according to claim 7, characterised in that the membrane (25) is advantageously an ultra-thin membrane of the type used in micro-dialysis, has hydrophobic properties and is preferably produced from silicone or PTFE.

9. Device according to any one of claims 7 and 8, characterized in that the filtering device is mounted directly in the container (5) for receiving eye prostheses, the membrane filter (25) and its protective grids (26) being held in their housing (28) by means of an elastic ring (29) which applies them to encircling lips (30).

10. Device according to claim 4, characterised in that the switch (3) actuates a timer which cuts off the supply of electricity after a predetermined period.

Fig.-1

Fig.3

Fig.4

EP 0 170 602 B1

Fig. 2

# Fig. 5